# EUROPEAN PATENT APPLICATION

(11) **EP 0 577 280 A1**
(43) Date of publication of application: **05.01.1994**
(21) Application number: 93304556.9
(22) Date of filing: 11.06.1993
(51) Int. Cl.: C07C 5/333, C07C 11/09, B01J 21/18

(54) **Process for the dehydrogenation of hydrocarbons using a carbonaceous catalyst**

(30) Priority: 18.06.1992 US 900977
(71) Applicant: EXXON RESEARCH AND ENGINEERING COMPANY, Florham Park, New Jersey 07932-0390 (US)
(72) Inventor: Kerby, Michael Charles, Jr., Baton Rouge, LA 70810 (US); Bearden, Roby, Jr., Baton Rouge, LA 70808 (US); Davis, Stephen Mark, Baton Rouge, LA 70817 (US)
(74) Representative: Somers, Harold Arnold

(57) **Abstract**

The present invention relates to a process for dehydrogenating C₂ - C₁₀ alkyl hydrocarbons by contacting the hydrocarbons with a carbonaceous catalyst at dehydrogenation conditions, which include temperatures in the range of about 400°C to about 1200°C. A preferred carbonaceous catalyst is a petroleum coke.

## Description

### FIELD OF THE INVENTION

The present invention relates to a novel process for the dehydrogenation of hydrocarbons by contacting said hydrocarbons at dehydrogenation conditions with a catalyst comprised of carbonaceous solids.

### BACKGROUND OF THE INVENTION

Transportation fuels, particularly motor gasoline, contains a relatively high level of aromatic components, such as benzene. These fuels, while relative high in octane number, are facing ever growing difficulty meeting ever stricter governmental environmental regulations with regard to emissions. This is primarily because of the high levels of aromatics. Consequently, there is much work being done to develop what has become known as "low emissions fuels". An important aspect of this work involves the substitution of non-aromatic components, having a relatively high octane value, for aromatic components of the fuel.

A class of non-aromatic components having relatively high octane value, which has been proposed for the production of low emissions fuels, are oxygenates. Non-limiting examples of preferred oxygenates for use in low emissions fuels include the unsymmetrical dialkyl ethers, particularly methyl *tert*-butyl ether (MTBE), ethyl *tert*-butyl ether (ETBE), and *tert*-amylmethyl ether (TAME). Conventional methods of manufacture of such ethers are typically based on liquid-phase reactions, such as the reaction of iso-butylene with methanol over cation-exchanged resins. This has created substantial demand for oxygenate building blocks, such as iso-butylene. Furthermore, other low carbon, or light, olefins are also in demand for the same reasons.

Low carbon number olefins, for example those having 2 to 10 carbon atoms, are typically obtained by the dehydrogenation of the corresponding paraffinic hydrocarbon. One method for light paraffin dehydrogenation is the so-called oxidative dehydrogenation process. In this process the light alkanes are reacted with oxygen over a suitably prepared mixed metal oxide catalyst to produce a mixture of olefin, water, COₓ, and unreacted paraffin. While high conversions combined with high olefin selectivities can be achieved, such a process has a number of disadvantages including loss of fuel value due to water and COₓ formation. Furthermore, process operations are relatively costly and there are industrial hazards associated with exothermic combustion reactions.

A more direct and preferred approach is direct dehydro-genation over a suitable catalyst to produce olefins and molecular hydrogen. This chemistry has recently received considerable interest, although high reaction temperatures in the range of 500-650°C are required to obtain a significant equilibrium yield (e.g., 15-65%) of olefin. Moreover, under these reaction conditions, light alkane hydrogenolysis, for example to methane, is a competing and undesirable reaction. Most catalysts studied to date have not shown very high selectivities for dehydrogenation versus hydrogenolysis, or they have suffered from rapid catalyst deactivation necessitating frequent regeneration. As a consequence, process economics have not been favorable. Consequently, incentives exist for catalysts which: have high selectivity for the production of olefins; have improved resistance to deactivation; and can be regenerated using simple procedures, such as air treatment.

### SUMMARY OF THE INVENTION

In accordance with the present invention, there is provided a process for dehydrogenating hydrocarbons. The process comprises contacting a feedstream containing hydrocarbons selected from the group consisting of one or more C₂ - C₁₀ non-aromatic hydrocarbons, and alkylated aromatic compounds wherein the alkyl group contains from about 2 to 10 carbon atoms; at dehydrogenation conditions; with a carbonaceous catalyst characterized as: (i) being a refractory material; and (ii) being comprised of at least 60 wt.% carbon; wherein said dehydrogenation conditions include temperatures from about 400 to 1200° C and pressures from about sub-atmospheric to about 50 atmospheres.

In preferred embodiments of the present invention, the carbonaceous material is selected from the group consisting of petroleum coke, activated carbon, soot, and coal char.

In other preferred embodiments of the present invention, the petroleum coke is selected from coke from a fluid coker process unit and coke from a delayed coker process unit.

In still other preferred embodiments of the present invention, the hydrocarbons in the hydrocarbon stream are primarily C₂ to C₆ alkanes.

### DETAILED DESCRIPTION OF THE INVENTION

Hydrocarbon streams which are dehydrogenated in accordance with the present invention are any which contain a substantial amount, preferably greater than about 90 wt.%, of C₂ to C₁₀ hydrocarbons. More preferred are the C₂ to C₁₀ alkanes; alkenes; alicyclic compounds, such as cyclohexane; alkylaryl compounds, wherein the alkyl group contains from about 2 to 10 carbon atoms, such as ethylbenzene; and naphtheno-aromatics, such as tetrahydronaphthalene. Particularly preferred are C₂ to C₆ hydrocarbons, and more particularly preferred are C₂ to C₅ hydrocarbons, especially the alkanes, iso-butane and iso-pentane. It is to be understood that derivatives of the above hydrocarbons may also be used in the practice of the present invention, such as alcohols, halides, carboxylic acids, and the like. Typical hydrocarbon streams which will be treated in accordance with the present invention are petroleum refinery streams. Non-limiting examples of such streams are the C₃ - C₅ streams which result from reforming, coking, hydrocracking, and fluid catalytic cracking.

The alkyl portion of the hydrocarbons of the feedstream are dehydrogenated by contacting the stream with an effective carbonaceous catalyst at dehydrogenation conditions. Effective carbonaceous catalysts which may be used in the practice of the present invention are those characterized as: (i) being a refractory material; and (ii) being comprised of at least 60 wt.% carbon, preferably 70 wt.% carbon, more preferably at least about 80 wt.% carbon, and most preferably at least about 85 wt.% carbon. The carbonaceous catalyst material will be used as received without the adding catalytic metals. That is the carbonaceous materials of the present invention are not used as supports for the addition of catalytic metals. Materials such as coal char will contain a substantial amount of mineral matter in the form of metallic oxides, sand carbonates, etc. This mineral matter will be substantially inert. By "refractory", we mean that the carbonaceous catalyst material will substantially maintain its structure at the dehydrogenation conditions employed. Non-limiting examples of preferred carbonaceous materials which can be used as catalysts of the present invention include petroleum coke; activated carbons; soot; coal chars; carbon blacks; poly-nuclear-aromatics, such as molecular moieties which contain at least four fused aromatic rings; and fullerenes, also sometimes referred to as buckminsterfullerenes, or Buckyballs.

Activated carbons are typically prepared by the destructive distillation of wood, nut shells, animal bones, etc. They are usually activated by heating to a temperature in excess of about 800° C with steam or carbon dioxide, which results in a porous internal structure. Carbon blacks are generally finely divided forms of carbon made by the incomplete combustion or thermal decomposition of natural gas or petroleum oil. The principal types of carbon blacks are channel black and furnace black. Coal char results from the pyrolysis of coal. Fullerenes are hollow, all carbon containing molecules that each have the carbon atoms arranged at the vertices of 12 pentagons, and differing numbers of hexagons. The most abundant species of fullerenes identified to date is the C₆₀ molecule that consists of carbon atoms located at the vertices of 12 pentagons and 20 hexagons arranged with the symmetry of an icosahedron. A Preferred carbonaceous catalyst material for the present invention is petroleum coke, particularly a petroleum coke which has been subjected to a high temperature burner in excess of about 900° C.

The average particle size of the carbonaceous catalyst is not critical, but it will generally range from about sub-micron size to about 500 microns. The particular particle size used in the practice of the present invention will be any suitable size for the reactor employed. For example, fluid-beds typically require an average particle size of about 50 to 250 microns.

In fluid coking, a heavy hydrocarbonaceous material, typically having a Conradson carbon content of at least about 5 wt.%, more typically from about 5 wt.% to 50 wt.%, is fed into a fluid coker unit which contains a coking zone comprised of a bed of hot fluidized refractory solid particles - typically coke particles. The fluidizing medium is usually steam. As the heavy hydrocarbonaceous material comes into the contact with the hot coke particles, it is cracked to lighter liquid and gaseous products, as well as coke, which is deposited on the fluidized coke particles. The coked coke particles are then passed to a burner where the coke in partially burned-off at a temperature less than about 700° C. A portion of the hot coke particles are recycled to the coking zone to maintain the heat of reaction. A preferred fluid coking process is one which contains a gasifier, or burner, which is operated at a temperature in excess of 900° C. The hot coke particles from the gasifier are typically not sent directly to the coking zone, but are first passed to a lower temperature heat removal zone to reduce their temperature prior to them being sent to the coking zone.

It is preferred to practice the present invention in a continuous operation wherein the hydrocarbon stream is introduced into a reactor containing the carbonaceous catalyst material. The reactor, of course, will be operated at dehydrogenation conditions. That is, from a temperature from about 400° C to about 1200° C, preferably from about 400° C to about 1000° C, and more preferably from about 400° C to about 800° C. Pressures will typically range from about sub-atmospheric to about 50 atmospheres, preferably from about 0.1 atmospheres to 5 atmospheres, and more preferably from about atmospheric pressure to about 3 atmospheres.

Any suitable reactor design may be used in the practice of the present invention. For example, a fixed-bed reactor may be used wherein the hydrocarbon stream is passed through a relatively dense bed of carbonaceous catalyst material. Preferred is fluidized-bed or a moving-bed reactor.

The general principle of operation of a continuous moving-bed process is that the catalyst is contained in an annular bed formed by spaced cylindrical screens within a reactor. The reactant stream, in this case the hydrocarbon stream, is processed through the catalyst bed, typically in an out-to-in radial flow. That is, it enters the reactor at the top and flows radially from the reactor wall through the annular bed of catalyst which is descending through the reactor, and passes into the cylindrical space created by said annular bed. It exits the bottom of the treating zone and can be collected, or passed, to further processing. The alkyl portion of the hydrocarbon components of the stream will react with the carbonaceous catalyst resulting in a product stream containing hydrogen and olefin product. The product stream can be collected overhead of the reactor where it can be passed to a fractionator for separation of the olefinic fraction from hydrogen. The annular moving-bed of catalyst will exit from the bottom section of the reaction zone and passed to a regeneration zone where it is heated to an effective temperature to regenerate the catalyst, usually by burning off any accumulated carbon. A stream of hot carbonaceous material is recycled to the reaction zone to maintain the reaction zone at dehydrogenation temperatures.

It will be understood that the present invention may be practiced by injecting the hydrocarbon stream into the transfer line of a fluid coker which carries hot coke from the burner to the coker, preferably to the scrubbing section of the coker. It is preferred that the transfer line be from a fluid coker which contains a gasifier which subjects the coke to temperatures in excess of about 900° C. The hydrocarbon stream can be injected directly into the transfer line and reacted with the hot coke particles, at temperatures suitable for dehydrogenation of hydrocarbons. The olefinic reaction products are collected from the scrubbing zone of the coker reaction vessel and sent downstream to fractionation. Further, instead of C₂ to C₁₀ hydrocarbons, methane can be reacted with the carbonaceous catalysts of the present invention to produce hydrogen and carbon. Steam can also be injected with the methane to produce hydrogen and carbon monoxide, instead of hydrogen and carbon.

The following examples are presented for illustrative purposes and should not be taken as limiting the invention in any way.

### Example 1 (Comparative)

A very low surface area (<1 m²/g) inert alumina solid, designated as Denstone (2.5 mL), available from Norton Chemical Process Products Co., was tested for iso-butane dehydrogenation. The Denstone was added to a quartz reactor tube approximately 1 cm in diameter and 70 cm long. The reactor tube was heated to a temperature of 650° C under flowing nitrogen (100 mL/min). The Denstone was chosen to provide an essentially purely thermal conversion reference. Upon reaching the dehydrogenation reaction temperature (650° C), the nitrogen was shut off and iso-butane was fed to the reactor tube at a rate of 44.4 mL/min, at 1 atmosphere and 1 second vapor residence time. The reaction products were sampled after 80 sec on stream and analyzed by use of a gas chromatograph. The iso-butane conversion was found to be 13.3 wt.%, the iso-butylene selectivity 55.2 wt.%, and the iso-butylene yield 7.3 wt.%.

### Example 2

The procedure of Example 1 was followed except that 2.5 mL of a carbon black designated ELFTX 5 GP-3205, available from Cabot Corp., and having a surface area of 62 m²/g was used instead of Denstone. The iso-butane conversion was found to be 23.5 wt.%, the iso-butylene selectivity 60.0 wt.%, and the iso-butylene yield 14.1 wt.%.

### Example 3

The procedure of Example 1 was followed except that 2.5 mL of a carbon black designated as Vulcan XC 72R, also available from Cabot Corp. and having a surface area of 242 m²/g, was used as the catalyst. The iso-butane conversion was found to be 42.1 wt.%, the iso-butylene selectivity 43.7 wt.%, and the iso-butylene yield 18.4 wt.%.

### Example 4

The procedure of Example 1 was followed except that 2.5 mL of a carbon black designated Monarch 800 CS-4384, also available from Cabot Corp, and having a surface area of 224 m²/g, was used as the catalyst. The iso-butane conversion was found to be 48.2 wt.%, the iso-butylene selectivity 64.5 wt.%, and the iso-butylene yield 31.1 wt.%.

### Example 5

The procedure of Example 1 was followed except that 2.5 mL of an activated carbon designated Darco G-60, available from American Norit Co., and having a surface area of 833 m²/g, was used as the catalyst. The iso-butane conversion was found to be 43.8 wt.%, the iso-butylene selectivity 61.1 wt.%, and the iso-butylene yield 26.8 wt.%.

### Example 6

The procedure of Example 1 was followed except that 2.5 mL of an activated carbon designated Raven-1025 and available from Columbia Carbon Inc., and having a surface area of 1194 m²/g, was used as the catalyst. The iso-butane conversion was found to be 72.9 wt.%, the iso-butylene selectivity 61.9 wt.%, and the iso-butylene yield 45.1 wt.%.

### Example 7

The procedure of Example 1 was followed except that 2.5 mL of an activated carbon designated Super Sorb and available from Amoco Corp. and having a surface area of about 3000 m²/g, was used as the catalyst. The iso-butane conversion was found to be 55.6 wt.%, the iso-butylene selectivity 73.2 wt.%, and the iso-butylene yield 40.7 wt.%.

### Example 8

The procedure of Example 1 was followed except that 2.5 mL of a carbon soot, enriched with about 5 wt.% fullerenes, and obtained from Texas Fullerene Co., and having a surface area of 256 m²/g, was used as the catalyst. The iso-butane conversion was found to be 32.0 wt.%, the iso-butylene selectivity 74.8 wt.%, and the iso-butylene yield 23.9 wt.%.

### Example 9

The procedure of Example 1 was followed except that 2.5 mL of coke from a fluid coker containing a burner which subjected the coke to temperatures in excess of 900° C was used as the catalyst. The coke had a surface area of 158 m²/g. The iso-butane conversion was found to be 55.6 wt.%, the iso-butylene selectivity 77.7 wt.%, and the iso-butylene yield 43.2 wt.%.

### Example 10

The procedure of Example 1 was followed except that 2.5 mL of coke from a fluid coker containing a burner which subjected the coke to temperatures less than about 700° C was used as the catalyst. The coke had a surface area of about 17 m²/g. The iso-butane conversion was found to be 13.2 wt.%, the iso-butylene selectivity 74.8 wt.%, and the iso-butylene yield 9.9 wt.%.

### Example 11

The procedure of Example 1 was followed except 2.5 mL of coke from a delayed coker and having a surface area of about 2 m²/g was used as the catalyst. The iso-butane conversion was found to be 22.0 wt.%, the iso-butylene selectivity 54.6 wt.%, and the iso-butylene yield 12.0 wt.%.

### Example 12

The procedure of Example 1 was followed except 2.5 mL of coal char obtained from the pyrolysis of Wyodak subbituminous coal was used as the catalyst. The coal char had a surface area of about 9 m²/g. The iso-butane conversion was found to be 30.3 wt.%, the iso-butylene selectivity 61.0 wt.%, and the iso-butylene yield 18.5 wt.%.

## Claims

1. A process for dehydrogenating hydrocarbons, which process comprises contacting a feedstream containing hydrocarbons selected from the group consisting of one or more C₂ - C₁₀ non-aromatic hydrocarbons, and/or alkylated aromatic compounds wherein the alkyl group contains from about 2 to 10 carbon atoms, and/or derivatives of the foregoing, at dehydrogenation conditions; with a carbonaceous catalyst characterized as: (i) being a refractory material; and (ii) being comprised of at least 60 wt.% carbon; wherein said dehydrogenation conditions include temperatures in a range of from about 400 to 1200°C and pressures in a range of from about sub-atmospheric to about 50 atmospheres.

2. The process of claim 1 wherein the carbonaceous catalyst contains at least 85 wt.% carbon.

3. The process of claim 1 or claim 2 wherein the carbonaceous catalyst is selected from carbonaceous materials comprising petroleum coke, coal char, soot, activated carbon, and carbon black.

4. The process of any one of claims 1 to 3 wherein the dehydrogenation reaction is operated at a temperature in a range of from about 400°C to 1000°C.

5. The process of any one of claims 1 to 4 wherein the reaction is conducted under pressures in a range of from about 0.1 atmospheres to about 5 atmospheres.

6. The process of any one of claims 1 to 5 wherein the dehydrogenation reaction is conducted in either a fluidized-bed reactor or a continuous moving-bed reactor.

7. The process of claim 6 wherein the reactor is a fluidized-bed reactor and the carbonaceous catalyst is selected from petroleum coke, coal char, soot, activated carbon, and carbon black.

8. The process of claim 6 or claim 7 wherein the catalyst is a petroleum coke from a fluid coker which has gasified the coke in a gasification zone operated at a temperature in excess of about 900°C.

9. The process of claim 8 wherein the feedstream is contacted with said petroleum coke in a transferline of the fluid coker.

10. The process of claim 9 wherein the hydrocarbon stream is a C₃ - C₅ refinery stream resulting from a refinery process.

11. The process of claim 10 wherein the refinery process is selected from reforming, coking, hydrocracking, and fluid catalytic cracking.
